# EUROPÄISCHE PATENTSCHRIFT

(11) **EP 3 063 261 B1**
(45) Veröffentlichungstag und Bekanntmachung des Hinweises auf die Patenterteilung: **30.11.2022**
(21) Anmeldenummer: 14799361.2
(22) Anmeldetag: 29.10.2014
(51) Int. Cl.: C12M 1/00, C12M 1/26, C12M 1/34, C12M 1/12

(54) **DECKEL FÜR ZELLKULTURBEHÄLTER**
COVER FOR A CELL-CULTURE CONTAINER
COUVERCLE POUR CONTENEUR DE CULTURE CELLULAIRE

(30) Priorität: 31.10.2013 DE 102013112049
(43) Veröffentlichungstag der Anmeldung: 07.09.2016
(73) Patentinhaber: Hamilton Bonaduz AG, 7402 Bonaduz (CH)
(72) Erfinder: JÄGER, Thomas, CH-7402 Bonaduz (CH); ETZOLD, Carsten, CH-7402 Bonaduz (CH); DEMARMELS, René, CH-7208 Malans (CH)
(74) Vertreter: Caspary, Karsten
(86) Internationale Anmeldenummer: PCT/EP2014/073197
(87) Internationale Veröffentlichungsnummer: WO 2015/063136

(56) Entgegenhaltungen:
- EP-A1- 2 141 224
- EP-A1- 2 251 407
- WO-A1-2009/009771
- WO-A1-2011/090781
- DE-A1- 4 207 346
- US-A- 5 350 080
- US-A- 5 848 622
- US-A1- 2009 152 744
- US-A1- 2011 207 170
- US-A1- 2011 223 076

## Beschreibung

Die vorliegende Erfindung betrifft einen Deckel für Zellkulturbehälter, wie sie üblicherweise zur industriemäßigen Kultivierung von adhärenten Zellen in Laboren der Pharma- bzw. Biotechnologieindustrie eingesetzt werden.

Auf dem Gebiet der Zellkultur bekannt sind Zellkulturbehälter, die in der Regel aus durchsichtigem Kunststoff, häufig als Wegwerf- oder Einwegbehälter, ausgebildet sind und über einen oder mehrere Schraubverschlüsse verfügen. Die Zellkulturbehälter werden dazu verwendet, um Zellen zu vermehren bzw. zu kultivieren und dabei das Wachstum zu beobachten. Dazu werden die zu kultivierenden Zellen zusammen mit einem geeigneten Nährmedium in den Behälter (oft in Flaschenform) hineingegeben, wo sie sich bei entsprechenden Umgebungsbedingungen vermehren können. Das Innere des Zellkulturbehälters umfasst ein Volumen, das deshalb als Kulturvolumen bezeichnet wird. Die Umgebungsbedingungen sind in der Regel eine Umgebungstemperatur von 37°C mit einem CO₂-Gehalt von 5 % sowie einer relativen Luftfeuchtigkeit (rH) von 95 %. Sobald ein gewisser Abdeckungsgrad der Oberfläche des Zellkulturbehälters mit den adhärenten Zellen erreicht ist (Konfluenzgrad), müssen die Zellen entweder für die weitere Verwendung geerntet oder auf mehrere neue Zellkulturbehälter aufgeteilt werden. In manchen Fällen ist es auch notwendig, während der Kultivierung das Nährmedium auszutauschen (cell maintenance). Für den Austausch von Nährmedium und/oder die Entnahme von adhärenten Zellen, gegebenenfalls mit oder ohne Nährmedium, wird hierin zusammenfassend der Begriff Fluid verwendet.

Bei kleinen Mengen von zu kultivierenden Zellen, d. h. im einfachsten Fall, werden die oben genannten Prozessschritte in der Zellkultur manuell unter Zuhilfenahme von Handpipetten unter einem Sterilabzug durch entsprechend ausgebildete technische Assistenten durchgeführt. D. h. die Deckel der Zellkulturbehälter werden per Hand abgeschraubt und nach Befüllung bzw. Entnahme auch wieder manuell verschlossen. Eigens dafür vorgesehene Schüttelmaschinen und andere Laborgeräte ersetzen bereits Teilprozessvorgänge, um nicht sämtliche Tätigkeiten manuell durchführen zu müssen.

Für größere Mengen von Zellkulturen sind im Markt vollautomatisierte Lösungen bekannt, die sämtliche auszuführenden Schritte mit mechanischen Robotern durchführen, wie z. B. 6-Achs-Roboter der Firmen TAP oder Kawasaki. Bei diesen Roboterlösungen können die-10180 P 4981 WO
selben Zellkulturbehälter wie bei der manuellen Bearbeitung verwendet werden, was die Kosten für eine Neuanschaffung von Zellkulturbehältern vermeidet. Nachteilig an den automatisierten Roboterlösungen ist jedoch, dass der Durchsatz und die Geschwindigkeit der Verarbeitung sehr begrenzt und eine sinnvolle Parallelisierung bzw. Verschachtelung der Prozessschritte kaum möglich ist.

Die derzeit im Einsatz befindlichen Zellkulturbehälter verfügen über Schraubdeckel bzw. -verschlüsse, die wahlweise mit oder ohne Belüftungsmembran ausgeführt sind, um einen gezielten CO₂-Austausch mit den Zellen im Inneren der Flasche zu gewährleisten. Ein wichtiger Aspekt bei diesen Belüftungs- oder Filtermembranen ist, dass die Porengröße der Membran 0,22 µm nicht überschreitet, damit die Membran noch als Sterilmembran angesehen werden kann.

Des Weiteren existieren noch Sonderbauformen von Zellkulturbehältern mit Durchstechmembranen, die beispielsweise als hydrophobe Filtermembranen ausgebildet sind, wie z. B. die von der Firma Greiner Bio-One angebotene Zellkulturflasche "AutoFlask". Derartige Zellkulturbehälter sind für Automatisierungsprozesse optimiert, mit einer Vielfalt von Zellkultur- und Liquid Handling Systemen kompatibel und weisen beispielsweise ein physikalische Oberflächenbehandlung für adhärente Zellen auf. Nachteilig an derartigen Produkten ist jedoch, dass der Benutzer genau auf die Bauform, die Größe und die Oberflächeneigenschaften eingeschränkt ist. Aus diesem Grund finden derartige Systeme bisher nur begrenzt Einsatz.

Die deutsche Patentanmeldung DE 10 2013 201 069 der Anmelderin ist auf eine Zellkulturanlage mit einer Fluid-Versorgungsschnittstelle und einem speziell dafür konzipierten Zellkulturbehälter gerichtet, wobei der Zellkulturbehälter eine Einfüll- oder/und Belüftungsöffnung, sowie mindestens eine von der Einfüll- oder/und Belüftungsöffnung gesondert ausgebildete Gegenkopplungsformation aufweist, welche zur Herstellung und zur Lösung eines Kopplungseingriffs mit einer entsprechenden Kopplungsformation der Fluid-Versorgungsschnittstelle ausgebildet ist. Diese Zellkulturanlage bzw. dieser Zellkulturbehälter ist ebenfalls speziell an die entsprechende Anlage bzw. Schnittstelle angepasst, weshalb die bisher verwendeten Standard-Zellkulturbehälter nur zum Teil oder sehr eingeschränkt verwendet werden können. Um ein ganzes Labor bzw. eine Fertigung mit derartigen neuartigen Zellkulturbehältern auszustatten, sind deshalb sehr hohe Investitionen erforderlich.

Die US 2011/223076 A1 offenbart ein Anschlussstück für ein Bioreaktorgefäß. Das Anschlussstück weist eine Mehrzahl von Anschlüssen auf, die zum einen zur Einführung von Fühlern oder Sonden und zum anderen zur Zuführung einer Probe geeignet sind, sowie eine Ventilanordnung für einen Reinigungsanschluss.

Die DE 42 07 346 A1 offenbart eine Steuervorrichtung für ein Ventil eines Rotationsfüllers für die Getränkeindustrie, die ein Ventil mit einem in einem Ventilgehäuse zwischen einer Öffnungs- und einer Schließstellung bewegbar gelagerten Ventilstößel und einen in diesem angeordneten Stößelmagneten aufweist. Weiterhin ist wenigstens ein außerhalb des Ventilgehäuses angeordneter Steuermagnet vorhanden, welcher bei Drehung des Rotationsfüllers eine mit dem Stößelmagneten wechselwirkende Kontaktstellung durchläuft. Zur Vereinfachung der Steuervorrichtung und zur Sicherung von Öffnungs- bzw. Schließstellung besteht wenigstens ein Teil des Ventilgehäuses aus einem magnetisierbaren Material, an welchem der Stößelmagnet nach Durchlaufen der Kontaktstelle haftet, und ein zum Steuermagneten nachgeordneter und umgekehrt magnetisch gepolter Trennmagnet ist angeordnet, der den Stößelmagnet löst, wodurch der Ventilstößel bistabil anordenbar ist. US5848622 offenbart einen Verschluss für ein Bioreaktorgefäß, bestehend aus einem Deckel (20) mit einem Verteilelement (24) sowie einem Ventilelement (28). Das Ventilelement (24) hat die Funktion, dass beim manuellen Öffnen des Deckels das Bioreaktorgefäß entlüftet wird.

Es ist daher die Aufgabe der vorliegenden Erfindung, eine technische Lehre für einen Deckel eines Zellkulturbehälters anzugeben, wobei der Deckel die nicht nur die oben beschriebenen Nachteile überwindet, sondern auch mit bisher verwendeten Standard-Zellkulturbehältern verwendet werden kann, günstig herzustellen ist, und einen einfachen Aufbau aufweist.

Diese Aufgabe wird durch den Gegenstand des Anspruchs 1 gelöst. Vorteilhafte Ausführungsformen sind in den abhängigen Ansprüchen beschrieben.

Erfindungsgemäß wird ein Deckel für einen Zellkulturbehälter bereitgestellt, der ein Kulturvolumen umschließt und eine Öffnung aufweist, mit einer Ventilanordnung, geeignet zum Befüllen des Zellkulturbehälters mit einem Fluid und/oder zum Abpumpen von Fluid aus dem Zellkulturbehälter, und einer Ausgleichsöffnung, die zur Belüftung des Zellkulturbehälters und/oder zum Druckausgleich während des Befüllens oder Abpumpens geeignet ist, wobei die Ventilanordnung zwischen einer Durchlassstellung und einer Sperrstellung magnetisch schaltbar und an eine Fluid-Versorgungsschnittstelle anschließbar und als berührungslos schaltbar mit magnetischen Elementen ausgebildet ist. Durch die Integration der Ventilanordnung in den Deckel bzw.

Verschluss können bereits vorhandene Zellkulturbehälter problemlos in einer automatisierten Zellkulturanlage verwendet werden. Die Umrüstung von einer manuellen Verarbeitung zu einer im Wesentlichen automatisierten Zellkulturanlage erfordert damit lediglich die Investitionen in neue Deckel für bereits vorhandene Zellkulturbehälter. Durch die in den Deckel bzw. Verschluss integrierte Ausgleichsöffnung entfällt die Anforderung nach einer weiteren Öffnung im Zellkulturbehälter. Ein ordnungsgemäßer CO₂ Austausch ist damit gewährleistet. Ebenso ist dadurch ein ausreichender Volumenstrom beim Befüllen bzw. Entleeren des Zellkulturbehälters gesichert. Zudem ist der Aufbau des Deckels einfach und seine Herstellung kostengünstig, insbesondere im Vergleich zur speziellen Herstellung eines gesamten Zellkulturbehälters für eine automatisierte Zellkulturanlage.

Durch die als berührungslos schaltbar ausgebildete Ventilanordnung lassen sich automatisierte Zellkultursysteme bzw. -anlagen mit einem mit dem erfindungsgemäßen Deckel ausgestatteten Zellkulturbehälter auf einfache Weise nutzen. Die berührungslose Schaltbarkeit erlaubt einen einfachen Aufbau sowie eine besonders einfache Handhabung, da keine Kabel oder sonstige Verbindungen mit dem Deckel verbunden werden müssen, um die Öffnung bzw. das Schließen der Ventilanordnung zu bewirken. Dies ermöglicht es insbesondere, eine automatisierte Zellkulturanlage auf einfache Weise zu reinigen, im Gegensatz zu Schaltmechanismen von Ventilanordnungen, die nur durch Berührung zu öffnen bzw. zu schließen sind und durch den komplexen Aufbau nur mit hohem Aufwand zu reinigen sind. Mit besonderem Vorteil ist die Ausgleichsöffnung wahlweise als sich öffnen lassend und/oder als Membran ausgebildet. Damit kann die Ausgleichsöffnung während des Befüll- oder Entleerungsvorgangs geöffnet oder geschlossen werden, je nachdem, ob der Volumenstrom erhöht oder herabgesetzt werden soll. Die Ausbildung als Membran, vorzugsweise hydrophobe Membran, ist für den CO₂ Austausch im Ruhezustand wichtig, d.h. dann, wenn keine Entnahme, Befüllung bzw. kein Abpumpen stattfindet. Auch kann die Ausgleichsöffnung wie z.B. eine derartige Membran beispielsweise durch ein Gitter vor Beschädigungen geschützt sein ohne Beeinträchtigungen in ihrer Funktion hinnehmen zu müssen. Sofern der Deckel im Wesentlichen eine zylindrische Form aufweist, kann die Ausgleichsöffnung auf der Mantelfläche oder auch auf der Stirnfläche des Deckels angeordnet sein.

Besonders bevorzugt sind die Ventilanordnung und die Öffnung des Zellkulturbehälters koaxial angeordnet. D.h. dass die Ventilanordnung bei einer Zylinderform des Deckels im Wesentlichen in der Mitte der Stirnfläche des Deckels angeordnet ist, also in Verlängerung der gemeinsamen Achse. Bei dieser Anordnung ist die Ausgleichsöffnung vorteilhafter Weise auf der Mantelfläche angeordnet.

Mit weiterem Vorteil ist die Ausgleichsöffnung derart gestaltet, dass sie bei nicht an die Fluid-Versorgungsschnittstelle angeschlossener Ventilanordnung geschlossen ist und bei an die Fluid-Versorgungsschnittstelle angeschlossener Ventilanordnung geöffnet ist. Dies entspricht einer Andockfunktion, nach der die Ausgleichsöffnung erst dann geöffnet ist, wenn die Ventilanordnung des Deckels in dem Gegenstück, d.h. dem Port der Fluid-Versorgungsschnittstelle angeordnet ist. Beispielsweise erfolgt dies mittels eines mechanischen Schiebemechanismus, der eine Rückstellfeder aufweist.

Vorteilhafterweise ist der Deckel mit der Öffnung des Zellkulturbehälters mittels einer Gewindeverbindung, einer Bajonettverschlussverbindung, einer Presssitzverbindung, einer Schnapp- oder Steckverbindung oder einer Kombination daraus lösbar verbindbar. Die Verbindung mittels Gewinde, also der klassische Schraubverschluss, wird die hauptsächlich eingesetzte Verbindungsmöglichkeit sein, weil die bisher verwendeten Zellkulturbehälter über eine Öffnung mit einem Schraubgewinde verfügen. Es wird an dieser Stelle bemerkt, dass die entsprechende Verbindung auch mit Dichtungen versehen sein kann.

Mit besonderem Vorteil umfasst die Ventilanordnung ein magnetisches Kugelventil, wobei auf der behälternahen Seite der Ventilanordnung am Ventilsitz ein Magnetelement und auf der behälterfernen Seite der Ventilanordnung eine Kugel aus magnetischem Material angeordnet ist. Das magnetische Kugelventil hat den Vorteil, dass es kaum Fläche verbraucht, einfach aufgebaut ist und die behälterferne Seite des Deckels mit der Kugel relativ einfach gereinigt werden kann und damit eine wesentliche Anforderung an ein automatisches Zellkultursystem erfüllt ist, nämlich Kontaminationen der Zellkulturen so weit wie möglich zu reduzieren. Wie weiter unten ausführlicher beschrieben erfolgt die Steuerung des Kugelventils durch die Steuerungseinheit innerhalb der Fluid-Versorgungsschnittstelle. In geschlossenem Zustand wirken die magnetischen Kräfte der Kugel und das Magnetelement am Ventilsitz derart, dass sich die entsprechenden Elemente anziehen und somit die Kugel relativ fest auf dem Ventilsitz aufsitzt und damit Fluid daran gehindert ist, in den Zellkulturbehälter hinein oder aus ihm heraus zu strömen. Die von der Steuerungseinheit der Fluid-Versorgungsschnittstelle angesteuerten magnetischen Elemente sind im angedockten Zustand derart in der Nähe der Kugel angeordnet und so dimensioniert, dass eine Aktivierung der magnetischen Elemente ein magnetisches Feld erzeugt, das die Kugel aus ihrer geschlossenen Position heraus bewegt und damit der Strömungsweg für das Fluid freigegeben ist.

Bevorzugterweise ist der Ventilsitz konisch oder halbkugelförmig ausgebildet. Damit wird im geschlossenen Zustand die Kugel wirksam auf dem Ventilsitz gehalten und kann nur mit einer entsprechenden Minimalkraft von dort wegbewegt werden. Grundsätzlich ist es allerdings auch möglich, dass der Ventilsitz flach ausgebildet ist. Eine Reinigung ist dann besonders effektiv möglich, da die Zwischenräume für die Reinigungsflüssigkeit besser zugänglich sind.

Vorteilhafte Weise umfasst der Ventilsitz ein thermoplastisches Kunststoffmaterial wie TPE, Silikon oder dergleichen, wobei das thermoplastische Kunststoffmaterial bevorzugt eine Shore A Härte von etwa 25 bis etwa 50 aufweist. Derartige Materialien verleihen dem Ventilsitz eine im Wesentlichen leicht verformbare Eigenschaft und führen damit zu einer verbesserten Dichtwirkung der Ventilanordnung. Möglich ist es dabei auch, dass der Ventilsitz aus unterschiedlichen Materialien durch Zwei-Komponenten-Spritzguss hergestellt wird.

Bevorzugt ist der Deckel mit Ausnahme der Ventilanordnung einstückig aus Kunststoff mittels Spritzguss hergestellt, und die Ventilanordnung ist im Presssitz darauf aufsteckbar. Spritzguss ist bei den einschlägigen Kunststoffen wie PP, PE, PVC und dergleichen eine bewährte, technisch ausgereifte und sehr preisgünstige Herstellungsvariante. Durch das einfache Aufstecken der Ventilanordnung ist eine einfache Konstruktion gegeben. Optional können einzelne Bestandteile der Ventilanordnung (bei Ausbildung als magnetisches Kugelventil alle bis auf die magnetische Kugel und das Magnetelement) ebenfalls aus Kunststoff im Spritzgussverfahren hergestellt sein, auch durch Spritzguss mit zwei oder mehr Komponenten. Diese lassen sich mit den anderen Bestandteilen und den Deckelelementen auf einfache Art und Weise zu dem erfindungsgemäßen Deckel montieren. Damit ist es auch möglich, den Deckel als Einmal- /Wegwerfartikel auszugestalten, um den Aufwand für die Wiederaufbereitung nach Verwendung zu vermeiden.

Mit weiterem Vorteil weist der Deckel als Ausgleichsöffnung eine weitere Ventilanordnung auf. Anstatt einer eigenen "einfachen" Ausgleichsöffnung kann eine weitere Ventilanordnung deren Funktion übernehmen. Zur Trennung der Ventilanordnungen kann beispielsweise ein Siphon verwendet werden.

Es ist erfindungsgemäß auch möglich, mehrere Ventilanordnungen in dem Deckel, vorzugsweise nebeneinander, anzuordnen. Damit kann den Anforderungen an die Reinheit bzw. die Vermeidung von Kontamination noch stärker Rechnung getragen werden, z.B. in dem man eine Ventilanordnung nur für das Einleiten und eine weitere Ventilanordnung nur für das Abpumpen bzw. Herausleiten verwendet.

Die Erfindung wird nachfolgend ausführlich unter Bezugnahme auf in den Zeichnungen dargestellte Ausführungsbeispiele erläutert. Es zeigen:
- Fig. 1: eine perspektivische Darstellung einer bevorzugten Ausführungsform des erfindungsgemäßen Deckels für einen Zellkulturbehälter;
- Fig. 2: eine perspektivische Querschnittsansicht des erfindungsgemäßen Deckels aus Fig. 1;
- Fig. 3: eine Querschnittsansicht des erfindungsgemäßen Deckels aus Fig. 1;
- Fig. 4: eine perspektivische Ansicht einer zweiten Ausführungsform des erfindungsgemäßen Deckels;
- Fig. 5: einen erfindungsgemäßen Deckeln gemäß der ersten Ausführungsform, aufgesetzt auf einen Zellkulturbehälter sowie angedockt an eine Fluid-Verbindungsschnittstelle eines Zellkultursystems;
- Fig. 6: eine perspektivische Ansicht eines Zellkulturbehälters mit einem Deckel gemäß einer weiteren Ausführungsform der vorliegenden Erfindung;
- Fig. 7: eine Vorderansicht des Deckels aus Fig. 6;
- Fig. 8: eine Querschnittansicht eines Deckel gemäß der in den Fig. 6 und 7 dargestellten weiteren Ausführungsform der vorliegenden Erfindung;
- Fig. 9: ein Detail einer perspektivischen Querschnittansicht des in Fig. 6 dargestellten Deckels mit Zellkulturbehälter;
- Fig. 10: eine Querschnittsansicht eines Details der Darstellung aus Fig. 5; und
- Fig. 11: eine schematische Darstellung, die beispielhaft die Interaktion zwischen dem erfindungsgemäßen Deckel und einem Zellkultursystem gemäß einer bevorzugten Ausführungsform der Erfindung.

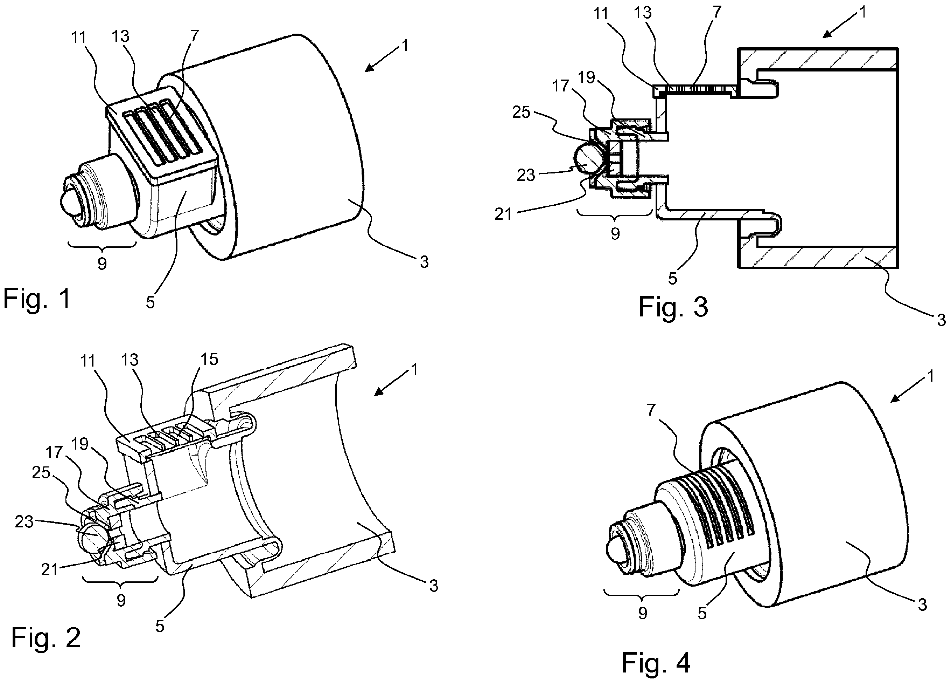

Fig. 1 zeigt in perspektivischer Darstellung eine bevorzugte erste Ausführungsform eines erfindungsgemäßen Deckels 1, der ein im Wesentlichen zylinderförmiges Deckelgehäuse 3 als Mantelfläche, ein behälterfernes Anschlussstück 5, an dessen Außenfläche sich eine Ausgleichsöffnung 7 befindet, sowie eine am anderen Ende des Anschlussstücks 5 angeordnete Ventilanordnung 9 aufweist. Die Ventilanordnung 9 umfasst ein magnetisches Kugelventil, das unter Bezugnahme auf die nachfolgenden Figuren näher erläutert wird. Die Ausgleichsöffnung 7 ist in dieser Ausführungsform zumindest teilweise mit einer Abdeckung 11 bedeckt, die mehrere Öffnungen 13 aufweist und eine gegebenenfalls darunter liegende Membran schützt. Die Ausgleichsöffnung 7 ist im Wesentlichen auf der Umfangs- bzw. Mantelfläche des Anschlussstücks 5 angeordnet. Der Durchmesser des Deckelgehäuses 3 ist so bemessen, dass das Anschlussstück 5 und die Ventilanordnung 9 in radialer Richtung nicht über die Mantelfläche hinausragen. Dies hat den Vorteil, dass bei einer Lagerung der erfindungsgemäßen Deckel 1 die empfindlichen Elemente wie die Ventilanordnung 9 oder die Ausgleichsöffnung 7 nicht bzw. in nur geringem Maße beschädigt werden können, wenn mehrere Deckel 1 zusammen, insbesondere nebeneinander, gelagert sind. In Fig. 1 ist auch gut erkennbar, dass die Ventilanordnung 9 eine relativ kleine Oberfläche aufweist, so dass die zu reinigende Fläche bei einem Fluidwechsel möglichst gering ist.

Fig. 2 zeigt eine Querschnittsansicht der Ausführungsform des erfindungsgemäßen Deckels 1 aus Fig. 1. In der dargestellten Ausführungsform ist das Innere des Deckelgehäuses 3 nicht mit einem Gewinde abgebildet, das auf einen entsprechenden, mit einem Gewinde versehenen Öffnungsstutzen oder -ansatz eines Zellkulturbehälters passt. Es versteht sich, dass neben den üblichen Gewindeverbindungen auch andere Arten von Deckel-Behälter-Verbindungen verwendet werden können wie z.B. Bajonettverschlussverbindungen, Presssitzverbindungen, Steckverbindungen oder dergleichen. Die Ausgleichsöffnung 7 ist neben einer (äußeren) Abdeckung 11 mit einer hydrophoben Membran 15 ausgestattet, die einen Austausch von CO₂ (oder anderen Gasen) zwischen dem Inneren des Zellkulturbehälters und der äußeren Umgebung insbesondere während der Lagerung des Zellkulturbehälters ermöglicht.

Die Ventilanordnung 9 umfasst am behälternahen Ende einen Stutzen oder Ansatz 19, der mit dem Anschlussstück 5 verbunden ist, sowie ein am behälterfernen Ende des Stutzens 19 angeordnetes Ventilgehäuse 17. In dem Ventilgehäuse 17 ist innen ein Magnetelement 21 angeordnet, und am behälterfernen Ende des Ventilgehäuses 17 ist ein Ventilsitz 25 angeordnet, der eine konisch nach innen zulaufende Form aufweist, so dass von außen eine magnetische Kugel 23 auf dem Ventilsitz 25 angeordnet ist. In der dargestellten Ausführungsform ist der Ventilsitz 25 aus einem thermoplastischen Kunststoffmaterial ausgebildet. Beispiele für ein derartiges Material sind die thermoplastischen Elastomere (TPE), die unter dem Handelsnamen Mediprene 500300M von der Firma Elasto oder unter dem Handelsnamen Thermolast M TM4RST von der Firma Kraiburg TPE erhältlich sind. Es versteht sich, dass der Fachmann optional auch ähnliche Kunststoffmaterialien oder Verbundwerkstoffe mit ähnlichen physikalischen Eigenschaften verwenden kann.

Das Magnetelement 21 ist in der dargestellten Ausführungsform als Permanentmagnet ausgebildet, wobei auch ferromagnetische Materialien geeignet sind. Die Kugel 23 ist ebenfalls aus magnetischem Material ausgebildet, also als Permanentmagnet oder als Ferromagnet, und sie weist eine magnetische Ausrichtung auf, die entgegengesetzt der magnetischen Polung des Magnetelements 21 ist, und zwar derart, dass im normalen Zustand, d.h. in dem Zustand, in dem der Deckel 1 auf einen Zellkulturbehälter aufgeschraubt bzw. aufgesetzt ist, die Kugel 23 und das Magnetelement 21 sich anziehen und die Ventilöffnung im Ventilsitz 25 ausreichend versperren, so dass kein Fluid aus dem Zellkulturbehälter heraus bzw. dort hinein gelangen kann.

Es ist anzumerken, dass als besondere Ausführungsform alle Elemente der Ventilanordnung 9 mit Ausnahme der magnetisch ausgebildeten Bauteile, dem Magnetelement 21 sowie der Kugel 23, integral mit den anderen Abschnitten des Deckels ausgebildet sein können, beispielsweise verkörpert durch ein einziges Spritzgussbauteil, in das das Magnetelement 21 von innen sowie die Kugel 23 von außen angeordnet sind.

Zwischen dem Anschlussstück 5 und dem Deckelgehäuse 3 ist eine Art Balgenelement angeordnet, das für einen gewissen Grad an Flexibilität der an dem Anschlussstück 5 angeordneten Elemente bezogen auf das Deckelgehäuse 3 bietet, wobei jedoch jederzeit eine Fluiddichtigkeit zwischen Anschlussstück 5 und Deckelgehäuse 3 gewährleistet ist. Das Balgenelement wird durch die Einstückigkeit des Deckelgehäuses 3 zusammen mit dem Anschlussstück 5 nicht beeinflusst. Mit anderen Worten, es ist möglich, einen erfindungsgemäßen Deckel 1 als ein integrales Spritzgussteil umfassend das Deckelgehäuse 3, Anschlussstück 5 und dazwischen liegendem Balgenelement auszubilden.

Fig. 3 zeigt eine Querschnittsdarstellung der unter Bezugnahme auf die Fig. 1 und 2 beschriebenen bevorzugten Ausführungsform des erfindungsgemäßen Deckels. Um Wiederholungen zu vermeiden, wird an dieser Stelle auf eine Beschreibung der Elemente verzichtet und auf die ausführliche Beschreibung zu Fig. 2 verwiesen.

Fig. 4 zeigt eine perspektivische Darstellung einer weiteren Ausführungsform des erfindungsgemäßen Deckels. Der Unterschied zu der in den Fig. 1 bis 3 dargestellten Ausführungsform liegt darin, dass die Ausgleichsöffnung 7 im Wesentlichen in die Mantelfläche des zylinderförmigen Anschlussstücks 5 integriert ist, indem die Öffnungen 13 sich schlitzartig in Umfangsrichtung parallel zueinander über einen vorbestimmten Winkelbereich des Umfangs des Anschlussstücks 5 erstrecken. Die Membran 15 ist dabei beispielsweise auf der Innenfläche des Anschlussstücks 5 derart angeordnet, so dass sie die Öffnungen 13 von innen abdeckt. Eine Abdeckung 11 wie in der ersten Ausführungsform ist in dieser Ausführungsform der Fig. 4 nicht vorhanden. Alle weiteren Elemente dieser zweiten Ausführungsform sind mit denen der unter Bezugnahme auf die Fig. 1 bis 3 beschriebenen Elemente der ersten Ausführungsform identisch.

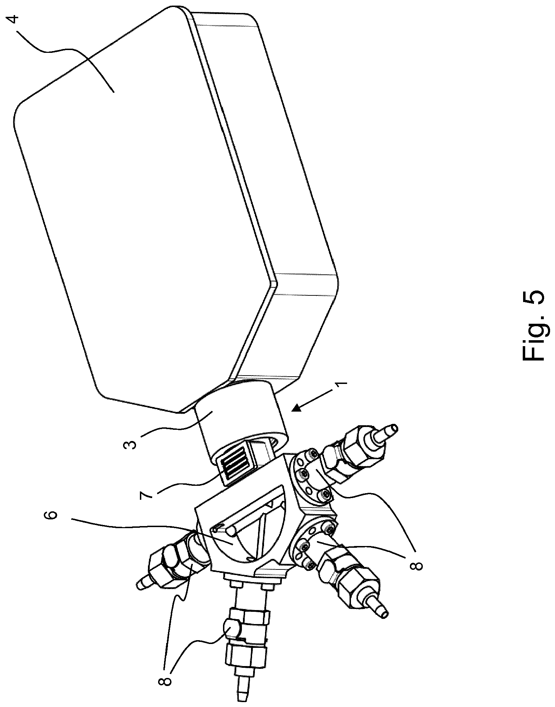

Fig. 5 zeigt eine perspektivische Darstellung eines erfindungsgemäßen Deckels gemäß der ersten Ausführungsform, der auf einen Zellkulturbehälter aufgeschraubt und an eine Fluid-Verbindungsschnittstelle eines Zellkultursystems angedockt ist. Der Deckel 1 ist im Inneren seines Deckelgehäuses 3 mittels eines Gewindes an einem entsprechenden Gewindestutzen einer Öffnung des Zellkulturbehälters in bekannter Weise befestigt. Auf der behälterfernen Seite ist die Ventilanordnung 9 (in Fig. 5 nicht sichtbar) an einer entsprechenden Anschlussformation einer Fluid-Versorgungsschnittstelle 6 eines Zellkultursystems angedockt.

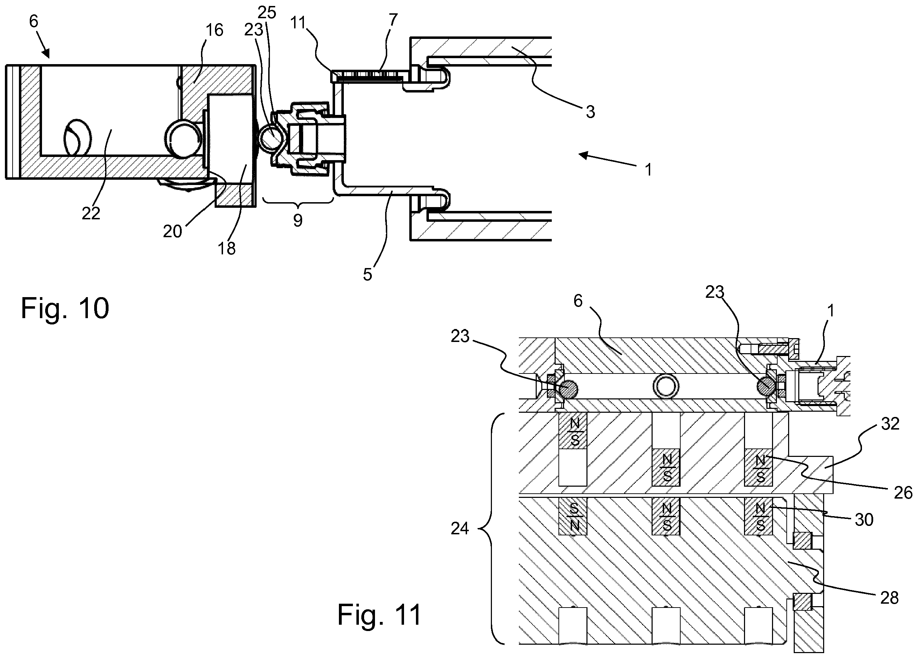

Die Fluid-Versorgungsschnittstelle 6 weist vier Anschlussformationen 8 auf, an die entsprechende Versorgungsbehälter für die Bevorratung von Fluiden bzw. zur Aufnahme von Fluiden im Betriebszustand des Zellkultursystems angeschlossen werden. In Fig. 5 nicht sichtbar dargestellt sind die Steuereinheit sowie die entsprechenden Signalmittel, um die Ventilanordnung 9 berührungslos zu steuern, d. h. insbesondere die magnetische Kugel vom Ventilsitz der Ventilanordnung 9 derart wegzubewegen, so dass ein Fluiddurchflusspfad je nach Beschaltung bzw. Steuerung der Fluid-Versorgungsschnittstelle 6 ermöglicht wird. Das Andockprinzip sowie die Steuerung innerhalb der Fluid-Versorgungsschnittstelle wird unten unter Bezugnahme auf die Fig. 10 und 11 näher beschrieben.

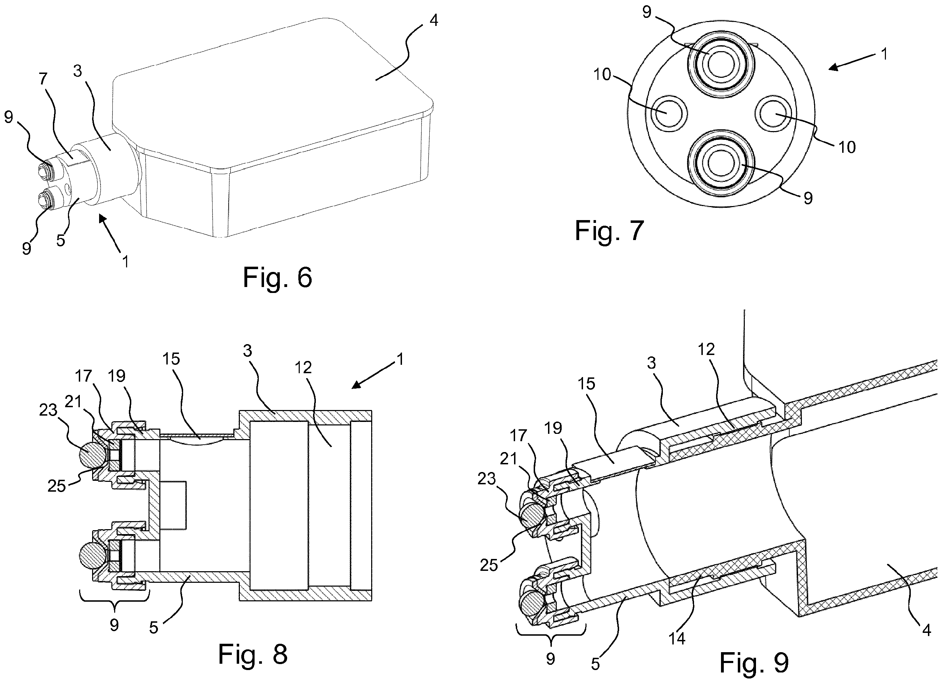

Fig. 6 zeigt eine perspektivische Ansicht eines Zellkulturbehälters mit einem Deckel gemäß einer weiteren Ausführungsform der vorliegenden Erfindung, bei der ein handelsüblich erhältlicher Zellkulturbehälter 4 mit einem erfindungsgemäßen Deckel 1 ausgestattet ist, der als wesentlicher Unterschied zu den zuvor beschriebenen Ausführungsformen nicht nur eine, sondern zwei Ventilanordnungen 9 aufweist. Die beiden Ventilanordnungen 9 sind parallel zueinander in axialer Richtung auf dem im Wesentlichen zylinderförmigen Anschlussstück 5 angeordnet, und zwar auf der behälterfernen Seite des Deckels 1. Die Ausgleichsöffnung 7 ist auf der Umfangsfläche des Anschlussstücks 5 angeordnet. Die Ventilanordnungen 9 sind im Wesentlichen identisch ausgebildet und im Wesentlichen symmetrisch bezüglich des Mittelpunkts der hier kreisförmigen Stirnfläche des Anschlussstücks 5 angeordnet.

Fig. 7 ist eine Vorderansicht des Deckels 1 gemäß der in Fig. 6 dargestellten zweiten Ausführungsform. Man erkennt, dass die Stirnfläche des Anschlussstücks 5 neben den beiden Ventilanordnungen 9 noch zwei Vertiefungen 10 aufweist, die zum automatischen Aufschrauben bzw. Abschrauben des Deckels 1 und/oder zum Zentrieren und damit Ausrichten des Deckels 1 bezüglich des Zellkulturbehälters 4 geeignet sind. Die Vertiefungen 10 sind auch dazu geeignet, um den Deckel 1 bezüglich der Fluid-Versorgungsschnittstelle 6 auszurichten. Es ist an dieser Stelle anzumerken, dass die Vertiefungen 10 keine Löcher in der Stirnfläche des Anschlussstücks 5 sind, sondern lediglich Ausnehmungen mit einer ausreichenden Tiefe, um die oben beschriebene Funktion zu gewährleisten.

Es versteht sich, dass erfindungsgemäß nicht exakt zwei Vertiefungen 10 in der Stirnfläche angeordnet sein müssen; es können auch mehr oder lediglich eine Vertiefung 10 vorhanden sein. Des Weiteren muss die Form der Vertiefung 10 nicht notwendigerweise kreisförmig sein. Sie kann rechteckig, quadratisch, sternförmig, polygonal oder andersartig ausgebildet sein.

Fig. 8 zeigt eine Querschnittsansicht eines Deckels gemäß der in den Fig. 6 und 7 dargestellten weiteren Ausführungsform der vorliegenden Erfindung. Wie bereits oben erläutert, weist der Deckel 1 in axialer Richtung zwei Ventilanordnungen 9 auf, die parallel zueinander ausgerichtet sind und im Wesentlichen identisch ausgebildet sind. Ihre Elemente sind identisch zu denen, die in Bezug auf die erste bevorzugte Ausführungsform beschrieben wurden. Aus diesem Grund wird auf eine Wiederholung an dieser Stelle verzichtet.

Man erkennt, dass sich anstatt einem Fluidströmungspfad bei angedocktem Deckel 1 zwei mögliche Fluidströmungspfade ausbilden können, je nachdem ob die entsprechenden Ventilanordnungen 9 geöffnet sind oder nicht. Es versteht sich, dass die Steuerung der beiden Ventilanordnungen 9 aus der in Fig. 8 dargestellten Ausführungsform unabhängig voneinander erfolgen kann. Insbesondere ist es auch möglich, dass, wenn eine der Ventilanordnungen 9 geöffnet ist und ein Fluid durchströmt, die andere Ventilanordnung als Druckausgleichsöffnung verwendet werden kann, um den Volumenstrom bzw. dessen Befüll- / Entleerrate zu steuern. Die Ausgleichsöffnung 7 ist in der hier dargestellten Ausführungsform durch die Membran 15 vorgesehen, die auf der Umfangsfläche des Anschlussstückes 5 angeordnet ist.

Im Gegensatz zu den zuvor dargestellten Ausführungsformen ist in dieser Ausführungsform des Deckels 1 das Deckelgehäuse 3 in seinem Innern mit einem Gewindeabschnitt 12 versehen, das zu einem entsprechenden Gewindeabschnitt auf dem Gewindestutzen des Zellkulturbehälters passt. Auch in dieser Ausführungsform erkennt man, dass die radiale Ausdehnung des mit den beiden Ventilanordnungen 9 versehenen Anschlussstücks 5 nicht über das Deckelgehäuse 3 hinausragt.

Fig. 9 zeigt ein Detail einer perspektivischen Querschnittansicht des in Fig. 6 dargestellten Deckels mit angeschlossenem Zellkulturbehälter. Wie bereits unter Bezugnahme auf Fig. 8 beschrieben, weist das Deckelgehäuse 3 des Deckels 1 auf seiner Innenseite einen Gewindeabschnitt 12 auf, der mit einem entsprechenden Gewindeabschnitt auf dem Gewindestutzen 14 des Zellkulturbehälters 4 zusammenpasst. In Fig. 9 ist der vollständig aufgeschraubte Zustand des Deckels 1 dargestellt, wobei man erkennt, dass das offene Ende des Gewindestutzens 14 des Zellkulturbehälters 4 an den stufenförmigen Anschlag zwischen Gehäusedeckel 3 und Anschlussstück 5 anstößt. Damit ist der Deckel 1 auf den Zellkulturbehälter 4 fest aufgeschraubt, wobei der zuvor beschriebene Anschlag eine zusätzliche Stabilisierung der Verbindung bereitstellt.

Es versteht sich, dass der erfindungsgemäße Deckel auch auf Zellkulturbehältern eingesetzt werden kann, die mehr als eine Öffnung zur Anbringung eines Deckels aufweisen. Der Hauptvorteil der vorliegenden Erfindung liegt jedoch darin, bereits existierende Zellkulturbehälter mit einer entsprechenden Schnittstelle auszurüsten, damit die bereits existierenden Zellkulturbehälter an ein automatisiertes Zellkultursystem angeschlossen werden können.

Weiterhin ist anzumerken, dass der erfindungsgemäße Deckel, insbesondere die Ventilanordnung eine Vorrichtung umfassen kann, die verhindert, dass vor allem im nicht angedockten Zustand Elemente der Ventilanordnung wie z.B. die magnetische Kugel im Falle eines magnetischen Kugelventils nicht verloren geht. Dabei kann es sich um eine Art aufsteckbaren bzw. abnehmbaren (Gitter-) Käfig, vorzugsweise aus Kunststoff, handeln, der um die Ventilanordnung(en) herum auf den Deckel aufgesetzt wird. Es ist dabei vorstellbar, dass dieser Käfig bei Andocken des Deckels an die Fluid-Versorgungsschnittstelle automatisch zur Seite schwenkt oder auch per Hand entfernt werden kann.

Unter Bezugnahme auf die Fig. 10 und 11 soll nun kurz die Funktionsweise der Interaktion eines erfindungsgemäßen Deckels mit einer Fluid-Versorgungsschnittstelle näher erläutert werden.

In Fig. 10 ist auf der rechten Seite der erfindungsgemäße Deckel 1 gemäß der ersten hier dargestellten Ausführungsform abgebildet; auf der linken Seite ist die Fluid-Versorgungsschnittstelle 6 zu erkennen, in die die Ventilanordnung 9 des Deckels 1 eingeschoben wird. Dabei wird die Ventilanordnung 9 in den Leerraum 18 eingeschoben, und zwar derart mittig, dass die magnetische Kugel 23 der Ventilanordnung 9 sich noch im Wesentlichen frei auf dem Ventilsitz 25 bewegen kann. Der Eingriff der Ventilanordnung 9 mit der Fluid-Versorgungsschnittstelle 6 kommt über die Stirnringfläche des Ventilsitzes 25 mit einer Gegendichtfläche 20 der Fluid-Versorgungsschnittstelle 6 zustande. Durch diese fluiddichte Anlage der Stirnringfläche auf der Gegendichtfläche 20 ist gewährleistet, dass kein Fluid außerhalb des Fluidströmungspfades gelangen kann. Bei geöffneter Ventilanordnung 9, d. h. wenn die Kugel 23 der Ventilanordnung 9 nicht mehr dichtend auf dem Ventilsitz 25 angeordnet ist, dann ergibt sich ein Fluidströmungspfad von dem Strömungsraum 22 der Fluid-Versorgungsschnittstelle 6 an der Kugel 23 vorbei in das Innere des Deckels 1 und damit in das Kulturvolumen des Zellkulturbehälters 4 (in Fig. 10 nicht dargestellt).

Anhand der Fig. 11 soll nun die Interaktion zwischen der Ventilanordnung 9 bzw. dem Deckel 1 und der Fluid-Versorgungsschnittstelle 6 mittels der Steueranordnung 24 erläutert werden. Die Steueranordnung 24 umfasst eine Vermittlungsanordnung 32 und eine Walze 28. Die drehbare Walze 28 weist einen (in Fig. 11 nicht dargestellten) elektromotorischen Antrieb zur Drehung um die Walzenachse und einer Mehrzahl von magnetischen Elemente 30 auf, die im dargestellten Ausführungsbeispiel als Permanentmagnete ausgeführt sind. Die magnetischen Elemente 30 sind vorzugsweise derart orientiert, dass ihre N-S-Polarisierungsrichtung mit einer von der Walzenachse ausgehenden radialen Richtung übereinstimmt. An den axialen Positionen der in Fig. 11 dargestellten magnetischen Elemente 30 können entlang des Umfangs der Walze 28 weitere magnetische Elemente 30 angeordnet sein.

Zwischen der Fluid-Versorgungsschnittstelle 6 und der Walze 28 ist in dem dargestellten Beispiel eine Vermittlungsanordnung 32 vorgesehen, um eine präzises Schalten der an der Fluid-Versorgungsschnittstelle 6 angedockten Ventilanordnungen zu ermöglichen. Für jede mögliche Ventilanordnung 9 ist auf der Vermittlungsanordnung 32 genau ein Magnetelement 26 vorgesehen, das hier als Permanentmagnet ausgebildet ist. Jedes Magnetelement 26 ist dabei in einem Kanal angeordnet, wobei das Magnetelement 26 zwischen einer walzennahen sowie in einer walzenfernen bzw. ventilanordnungsnäheren Stellung verschieblich gelagert ist.

Die Magnetelemente 26 sind dabei derart ausgewählt, dass das von ihnen ausgehende und auf die Kugeln 23 der entsprechenden Ventilanordnung 9 wirkende Magnetfeld zumindest in der walzenfernen Stellung stärker ist als das von den Magnetelemente 21 am Ventilsitz 25 ausgehende und auf die Kugel 23 wirkende Magnetfeld. Weiter sind die Magnetelemente 26 vorzugsweise längs ihrer Verlagerungsachse polarisiert angeordnet, etwa derart, das ein Pol wie der Nordpol zu der jeweils zugeordneten Ventilanordnung hinweist und der jeweils entgegengesetzte Pol, also der Südpol, zur Walze 28 hin zeigt.

Die Vermittlungsanordnung 32 ist bevorzugt derart angeordnet, dass die Magnetelemente 26 in den Kanälen durch die durch die Schwerkraft in ihre walzennähere Stellung vorgespannt sind, in der sich beispielhaft die beiden in Fig. 11 rechts angeordneten Magnetelemente befinden.

Durch entsprechende Orientierung der magnetischen Elemente 30 können bei Annäherung dieser magnetischen Elemente 30 an die Magnetelemente 26 der Vermittlungsanordnung 32 die Magnetelemente 26 durch die von den magnetischen Elementen 30 ausgehenden Magnetfelder von ihrer walzennäheren Stellung in ihre walzenferne bzw. ventilanordnungsnähere Stellung verlagert werden, etwa durch Gegenüberliegen von Polen gleicher Orientierung, also von sich abstoßenden Polen. In gleicher Weise werden sich die magnetischen Elemente 30 und die Magnetelemente 26 bei entgegengesetzt angeordneten Polen anziehen und damit zusätzlich zur Schwerkraft eine Anziehungskraft ausüben.

Bei Annäherung eines Magnetelements 26 der Vermittlungsanordnung 32 an die entsprechende Ventilanordnung wird die magnetische Kugel 23 durch das in seiner ventilanordnungsnäheren Stellung befindliche Magnetelement 26 stärker angezogen als durch das Magnetelement 21 der eigenen Ventilanordnung 9. Die Kugel 23 bewegt sich daher aus seiner Sperrstellung in die Öffnungsstellung, wobei der Fluiddurchlass durch die Ventilanordnung freigegeben wird. Man erkennt, dass die behälterferne Seite der Ventilanordnung 9 im Wesentlichen lediglich auf der Stirnseite, also dort wo die Kugel 23 angeordnet ist, gereinigt werden muss, wenn die Fluid-Versorgungsschnittstelle 6 sich im Reinigungsmodus befindet.

Alternativ zu der oben beschriebenen Ausgestaltung der Steuereinheit 24 können auch Elektromagnete als Aktoren verwendet werden, um das Öffnen und Schließen der Ventile zu bewirken. Schließlich sei auch erwähnt, dass anstatt von magnetischen Kugelventilen in der Ventilanordnung des erfindungsgemäßen Deckels auch piezoelektrische Ventile eingesetzt werden können oder andere dem Fachmann bekannte Ventile, die insbesondere auf der behälterfernen Seite leicht zu reinigen sind, um Kontaminationen im automatisierten Zellkultursystem soweit wie möglich zu vermeiden.

Mit dem erfindungsgemäßen Gegenstand wird ein Deckel für einen Zellkulturbehälter bereitgestellt, der mit bisher verwendeten Standard-Zellkulturbehältern verwendet werden kann, günstig herzustellen ist, und einen einfachen Aufbau aufweist.

## Patentansprüche

1. Deckel (1) für einen Zellkulturbehälter (4), der ein Kulturvolumen umschließt und eine Öffnung aufweist, mit
einer Ventilanordnung (9), geeignet zum Befüllen des Zellkulturbehälters (4) mit einem Fluid und/oder zum Abpumpen von Fluid aus dem Zellkulturbehälter (4), **dadurch gekennzeichnet, dass**
er eine Ausgleichsöffnung (7) aufweist, die zur Belüftung des Zellkulturbehälters (4) und/oder zum Druckausgleich während des Befüllens oder Abpumpens geeignet ist, wobei die Ventilanordnung (9) zwischen einer Durchlassstellung und einer Sperrstellung magnetisch schaltbar und an eine Fluid-Versorgungsschnittstelle (6) anschließbar und als berührungslos schaltbar mit magnetischen Elementen ausgebildet ist.

2. Deckel (1) nach Anspruch 1, **dadurch gekennzeichnet, dass** die Ausgleichsöffnung (7) wahlweise als sich öffnen lassend und/oder als Membran (15) ausgebildet ist.

3. Deckel (1) nach einem der vorhergehenden Ansprüche, **dadurch gekennzeichnet, dass** die Ventilanordnung (9) und die Öffnung des Zellkulturbehälters (4) koaxial angeordnet sind.

4. Deckel (1) nach einem der vorhergehenden Ansprüche, **dadurch gekennzeichnet, dass** die Ausgleichsöffnung (7) derart gestaltet ist, dass sie bei nicht an die Fluid-Versorgungsschnittstelle (6) angeschlossener Ventilanordnung (9) geschlossen ist und bei an die Fluid-Versorgungsschnittstelle (6) angeschlossener Ventilanordnung (9) geöffnet ist.

5. Deckel (1) nach einem der vorhergehenden Ansprüche, **dadurch gekennzeichnet, dass** der Deckel (1) mit der Öffnung des Zellkulturbehälters (4) mittels einer Gewindeverbindung, einer Bajonettverschlussverbindung, einer Presssitzverbindung, einer Schnapp- oder Steckverbindung oder einer Kombination daraus lösbar verbindbar ist.

6. Deckel (1) nach einem der vorhergehenden Ansprüche, **dadurch gekennzeichnet, dass** die Ventilanordnung (9) ein magnetisches Kugelventil umfasst, wobei auf der behälternahen Seite der Ventilanordnung (9) am Ventilsitz (25) ein Magnetelement (21) und auf der behälterfernen Seite der Ventilanordnung (9) eine Kugel (23) aus magnetischem Material angeordnet ist.

7. Deckel (1) nach Anspruch 6, **dadurch gekennzeichnet, dass** der Ventilsitz (25) ein thermoplastisches Kunststoffmaterial wie TPE, Silikon oder dergleichen aufweist.

8. Deckel (1) nach Anspruch 7, **dadurch gekennzeichnet, dass** das thermoplastische Kunststoffmaterial eine Shore A Härte von etwa 25 bis etwa 50 aufweist.

9. Deckel (1) nach einem der Ansprüche 6 bis 8, **dadurch gekennzeichnet, dass** der Ventilsitz (25) konisch oder halbkugelförmig ausgebildet ist.

10. Deckel (1) nach einem der vorhergehenden Ansprüche, **dadurch gekennzeichnet, dass** er mit Ausnahme der Ventilanordnung (9) einstückig aus Kunststoff mittels Spritzguss hergestellt ist, und dass die Ventilanordnung (9) im Presssitz darauf aufsteckbar ist.

11. Deckel (1) nach Anspruch 10, **dadurch gekennzeichnet, dass** er als Einmal- /Wegwerfartikel ausgebildet ist.

12. Deckel (1) nach einem der vorhergehenden Ansprüche, **dadurch gekennzeichnet, dass** er als Ausgleichsöffnung (7) eine weitere Ventilanordnung (9) aufweist.

13. Zellkulturbehälter (4), der ein Kulturvolumen umschließt und eine Öffnung aufweist, mit einem Deckel (1) nach einem der vorhergehenden Ansprüche.

## Claims

1. Cover (1) for a cell-culture container (4), which encloses a culture volume and has an opening, with
a valve arrangement (9), suitable for filling the cell-culture container (4) with a fluid and/or for pumping fluid out of the cell-culture container (4),
**characterized in that**
it has a compensation opening (7), which is suitable for aerating the cell-culture container (4) and/or for pressure compensation during filling or pumping out, wherein the valve arrangement (9) is formed magnetically switchable between an outlet position and a blocked position and connectable to a fluid supply interface (6) and contactlessly switchable with magnetic elements.

2. Cover (1) according to claim 1, **characterized in that** the compensation opening (7) is selectively formed as openable and/or as a membrane (15).

3. Cover (1) according to one of the preceding claims, **characterized in that** the valve arrangement (9) and the opening of the cell-culture container (4) are arranged coaxial.

4. Cover (1) according to one of the preceding claims, **characterized in that** the compensation opening (7) is configured in such a way that it is closed when the valve arrangement (9) is not connected to the fluid supply interface (6) and is opened when the valve arrangement (9) is connected to the fluid supply interface (6).

5. Cover (1) according to one of the preceding claims, **characterized in that** the cover (1) is detachably connectable to the opening of the cell-culture container (4) by means of a threaded connection, a bayonet connection, a press-fit connection, a snap-on or plug-in connection or a combination thereof.

6. Cover (1) according to one of the preceding claims, **characterized in that** the valve arrangement (9) comprises a magnetic ball valve, wherein a magnetic element (21) is arranged on the valve seat (25) on the side of the valve arrangement (9) near the container and a ball (23) made of magnetic material is arranged on the side of the valve arrangement (9) far away from the container.

7. Cover (1) according to claim 6, **characterized in that** the valve seat (25) has a thermoplastic plastic material such as TPE, silicone or the like.

8. Cover (1) according to claim 7, **characterized in that** the thermoplastic plastic material has a Shore A hardness of approximately 25 to approximately 50.

9. Cover (1) according to one of claims 6 to 8, **characterized in that** the valve seat (25) is formed conical or hemispherical.

10. Cover (1) according to one of the preceding claims, **characterized in that**, with the exception of the valve arrangement (9), it is produced in one piece from plastic by means of injection moulding, and **in that** the valve arrangement (9) is attachable to it with a press-fit.

11. Cover (1) according to claim 10, **characterized in that** it is formed as a single-use/disposable article.

12. Cover (1) according to one of the preceding claims, **characterized in that** it has a further valve arrangement (9) as compensation opening (7).

13. Cell-culture container (4), which encloses a culture volume and has an opening, with a cover (1) according to one of the preceding claims.

## Revendications

1. Couvercle (1) pour un contenant de culture cellulaire (4), qui renferme un volume de culture et présente un orifice, avec
un ensemble formant soupape (9) adapté pour remplir le contenant de culture cellulaire (4) d'un fluide et/ou pour évacuer par pompage du fluide hors du contenant de culture cellulaire (4),
**caractérisé en ce que**
il présente un orifice de compensation (7) qui est adapté pour l'aération du contenant de culture cellulaire (4) et/ou pour la compensation de pression au cours du remplissage ou de l'évacuation par pompage, dans lequel l'ensemble formant soupape (9) peut être commuté magnétiquement entre une position de passage et une position de blocage et peut être raccordé à une interface d'alimentation en fluide (6) et est réalisé avec des éléments magnétiques de manière commutable sans contact.

2. Couvercle (1) selon la revendication 1, **caractérisé en ce que** l'orifice de compensation (7) est réalisé au choix comme pouvant être ouvert et/ou comme une membrane (15).

3. Couvercle (1) selon l'une quelconque des revendications précédentes, **caractérisé en ce que** l'ensemble formant soupape (9) et l'orifice du contenant de culture cellulaire (4) sont disposés de manière coaxiale.

4. Couvercle (1) selon l'une quelconque des revendications précédentes, **caractérisé en ce que** l'orifice de compensation (7) est configuré de telle manière qu'il est fermé lorsque l'ensemble formant soupape (9) n'est pas raccordé à l'interface d'alimentation en fluide (6) et est ouvert lorsque l'ensemble formant soupape (9) est raccordé à l'interface d'alimentation en fluide (6).

5. Couvercle (1) selon l'une quelconque des revendications précédentes, **caractérisé en ce que** le couvercle (1) peut être relié de manière amovible à l'orifice du contenant de culture cellulaire (4) au moyen d'une liaison par filetage, d'une liaison par fermeture à baïonnette, d'une liaison par ajustement serré, d'une liaison par déclic ou par enfichage ou d'une combinaison de celles-ci.

6. Couvercle (1) selon l'une quelconque des revendications précédentes, **caractérisé en ce que** l'ensemble formant soupape (9) comprend une soupape sphérique magnétique, dans lequel un élément magnétique (21) est disposé sur le côté à proximité du contenant de l'ensemble formant soupape (9) sur le siège de soupape (25) et une sphère (23) composée d'un matériau magnétique est disposée sur le côté éloigné du contenant de l'ensemble formant soupape (9).

7. Couvercle (1) selon la revendication 6, **caractérisé en ce que** le siège de soupape (25) présente un matériau synthétique thermoplastique tel que le TPE, le silicone ou similaire.

8. Couvercle (1) selon la revendication 7, **caractérisé en ce que** le matériau synthétique thermoplastique présente une durée Shore A d'environ 25 à environ 50.

9. Couvercle (1) selon l'une quelconque des revendications 6 à 8, **caractérisé en ce que** le siège de soupape (25) est réalisé de manière conique ou en forme de demi-sphère.

10. Couvercle (1) selon l'une quelconque des revendications précédentes, **caractérisé en ce qu'**il est fabriqué, à l'exception de l'ensemble formant soupape (9), d'un seul tenant à partir de matière synthétique au moyen d'un moulage par injection, et que l'ensemble formant soupape (9) peut être emboîté sur celui-ci avec un ajustement serré.

11. Couvercle (1) selon la revendication 10, **caractérisé en ce qu'**il est réalisé en tant qu'article à usage unique/jetable.

12. Couvercle (1) selon l'une quelconque des revendications précédentes, **caractérisé en ce qu'**il présente en tant qu'orifice de compensation (7) un autre ensemble formant soupape (9).

13. Contenant de culture cellulaire (4), qui renferme un volume de culture et présente un orifice, avec un couvercle (1) selon l'une quelconque des revendications précédentes.
